# EUROPEAN PATENT APPLICATION

(11) **EP 4 779 647 A1**
(43) Date of publication of application: **22.07.2026**
(21) Application number: 24865804.9
(22) Date of filing: 10.09.2024
(51) Int. Cl.: G16H 30/40, G16H 30/20, G06T 3/60, G06T 7/30, G06T 17/20, G06T 5/80, G06T 5/73, G06T 3/4053, G06T 5/70, A61B 90/00

(54) **GUIDE SYSTEM FOR CORRECTING ROTATIONAL ALIGNMENT OF FEMUR USING ARTIFICIAL INTELLIGENCE IMAGE PROCESSING AND METHOD THEREOF**

(30) Priority: 14.09.2023 KR 20230122309
(71) Applicant: Kyungpook National University Industry-Academic Cooperation Foundation, Daegu 41566 (KR); Kyungpook National University Hospital, Jung-gu Daegu 41944 (KR)
(72) Inventor: OH, Changwug, Daegu 42028 (KR); LEE, JInhan, Daegu 41950 (KR); KIM, Joonwoo, Daegu 42231 (KR); PARK, Gyeonghyeon, Seoul 04114 (KR)
(74) Representative: Jung, Minkyu
(86) International application number: PCT/KR2024/013684
(87) International publication number: WO 2025/058365

(57) **Abstract**

An embodiment of the present invention provides a guide system for correcting rotational alignment of a femur using artificial intelligence image processing and a method thereof, the guide system comprising: an image capturing device for photographing an affected side and a healthy side of a patient; an image display device for displaying an affected side image including a femur in which an implant photographed by the image photographing device is combined, and a healthy side image including a normal femur; and a user device for obtaining an affected side image and a healthy side image from the affected side image and the healthy side image, matching the affected side image to the healthy side image based on the femur through artificial intelligence image processing, and calculating a rotation angle difference between a fractured femur in the affected side image and a normal femur in the healthy side image, thereby guiding rotational alignment correction of the affected side.

## Description

### Technical Field

The present invention relates to a guide system and a method for correcting rotational alignment of a femur using artificial intelligence image processing, and more specifically, to a guide system and a method for correcting rotational alignment of a femur through artificial intelligence image processing for a medical image used in an operating room.

### Background Art

In general, patients with femur fractures due to factors such as traffic accidents or industrial accidents account for a large proportion of all fracture patients. However, there are a number of problems such as nonunion or delayed union due to misalignment. Therefore, there is a need for an accurate femoral rotation alignment correction system that solves such a problem.

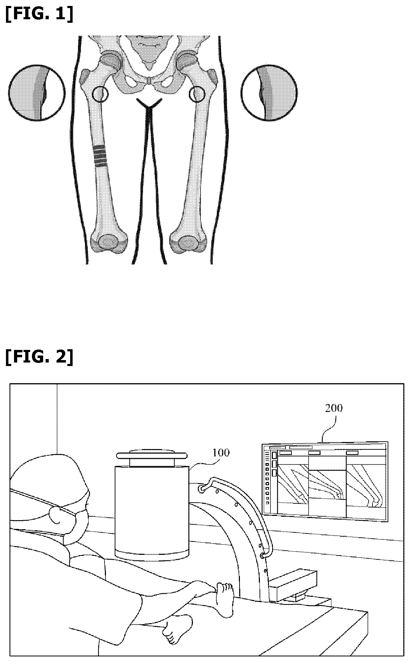

FIG. 1 is a view simulating images of the healthy side and the affected side in conventional femoral fracture treatment surgery. Existing femoral fracture surgery is performed while a number of mobile fluoroscopic images (hereinafter referred to as C-arm images) are taken. In order to check the proper rotation alignment, it is currently determined by the C-arm image. However, since the c-arm image only shows a two-dimensional image, it is difficult to know a three-dimensional rotation image. For this reason, there are many difficulties in accurate anatomical reduction.

In addition, radiation exposure of patients and medical staff becomes a serious problem due to multiple imaging. In addition, CT imaging after surgery is essential to check whether the alignment is accurate, and there is a problem in that discomfort and cost increase due to the high possibility of reoperation.

### Detailed Description

### Technical Problem

A technical object to be achieved by the present invention is to provide a guide system and a method for correcting the rotational alignment of the femur, which improve the quality of C-arm images used in an operating room and guide the correction of the rotational alignment by comparing the affected side and the healthy side using artificial intelligence image processing.

The technical problems to be achieved by the present disclosure are not limited to the technical problems mentioned above, and other technical problems not mentioned will be clearly understood by those skilled in the art to which the present disclosure pertains from the following description.

### Technical Solution

According to an aspect of the present disclosure, there is provided a guide system for correcting rotation alignment of a femur using artificial intelligence image processing, the guide system including: an image capturing device configured to capture an affected side and a healthy side of a patient; an image display device configured to display an affected side image including a femur in which an implant captured by the image capturing device is combined and a healthy side image including a normal femur; and a user device configured to obtain an affected side image and a healthy side image from the affected side image and the healthy side image, match the affected side image to the healthy side image based on the femur through artificial intelligence image processing to calculate a rotation angle difference between a fractured femur in the affected side image and a normal femur in the healthy side image, and guide rotation alignment correction of the affected side.

In an embodiment of the present invention, the image capturing device is a C-arm using an X-ray, the user device photographs the affected side image and the healthy side image displayed on the image display device or acquires the affected side image and the healthy side image through communication, and the user device includes: an image acquisition unit that acquires the affected side image and the healthy side image from the affected side image and the healthy side image, respectively; an image quality improvement unit that removes an implant coupled to a femur from the affected side image acquired through artificial intelligence image processing and improves the quality of an image including clarity thereof; an image registration unit that overlays the affected side image and the healthy side image, matches the size and position of the femur, and calculates an angle difference of rotation alignment; and a result display unit 340 that displays a result of the alignment by the image registration unit so that a user can recognize the result.

In an embodiment of the present disclosure, the user device is an MR-based (AR/VR) device, and may extract femur from the affected side image and the healthy side image displayed on the image display device using artificial intelligence image processing, configure a 3D model, and then perform 3D model matching using the 3D model.

According to another aspect of the present disclosure, there is provided a guide method for correcting rotation alignment of a femur using artificial intelligence image processing, the guide method including: acquiring, by an image acquisition unit of a user device, an affected side image and a healthy side image from an affected side image and a healthy side image by an image capturing device; removing, by an image quality improvement unit, an implant from the affected side image through artificial intelligence image processing; improving, by the image quality improvement unit, quality by imaging the affected side image from which the implant is removed; matching, by an image registration unit, the affected side image from which the implant is removed with the healthy side image; and displaying, by a result display unit, a result of a matching result including an angle difference between rotation alignment of the affected side femur and the healthy side femur calculated as a result of the registration.

In an embodiment of the present disclosure, the removing of the implant in the affected side image may include restoring image distortion and noise generated when the image quality improvement unit obtains the affected side image and the healthy side image through artificial intelligence image processing, separating the femur and the implant in the affected side image and removing the implant through artificial intelligence image processing, and restoring an original femur image to a region from which the image quality improvement unit has removed the implant.

In an embodiment of the present invention, the imaging of the image of the affected side to improve the quality may include: performing high resolution processing to improve resolution of the image of the affected side from which the implant is removed; performing a high sharpness operation to improve sharpness of the image of the affected side from which the implant is removed; and performing transparency processing for comparison with the image of the affected side or the healthy side on which the high resolution processing and the high sharpness operation are performed.

In an embodiment of the present invention, the matching of the healthy side image and the imaged affected side image may include: finding a femoral head and a femoral shaft in the healthy side image and the imaged affected side image, respectively; matching the imaged affected side image to the healthy side image with a scale of the femoral head and superimposing the same according to a position between the femoral bones; and calculating an angle difference between rotation alignment of the femoral head of the healthy side and the femoral head of the affected side.

### Advantageous Effect

According to an embodiment of the present invention, it is possible to provide a guide system and a method for correcting the rotational alignment of the femur that guides the correction of the rotational alignment capable of converting a photographed C-arm monitor image into an original form without distortion and noise through artificial intelligence image processing technology that improves readability by extracting only the femur from a C-arm image.

In addition, it is possible to provide a precision medical service that improves the surgical result of a femoral fracture surgery and enables rapid recovery by correcting and guiding the rotation alignment of the femur, which is difficult to correct in the operating room, using an artificial intelligence image processing system.

It should be understood that the effects of the present invention are not limited to the above-described effects, and include all effects that can be inferred from the detailed description of the present invention or the configuration of the invention described in the claims.

### Brief Description of Drawings

FIG. 1 is a view simulating images of the healthy side and the affected side in conventional femoral fracture treatment surgery.
FIG. 2 is a diagram for explaining a guide system for correcting rotational alignment of a femur using artificial intelligence image processing according to an embodiment of the present invention.
FIG. 3 is a block diagram illustrating a guide system for correcting rotational alignment of a femur using artificial intelligence image processing according to an embodiment of the present invention.
FIG. 4 illustrates an example of a user device in which an image obtained by photographing an image displayed on a monitor is displayed.
FIG. 5 is a diagram illustrating a result of registration displayed on a user device.
FIG. 6 shows an AR or VR device as another example of a user device.
FIG. 7 is a flowchart illustrating a guide method for correcting rotation alignment of a femur using artificial intelligence image processing according to an embodiment of the present invention.
FIG. 8 illustrates an example of a monitor that displays a C-arm image.
FIG. 9 shows an image of an affected side obtained by photographing an image of the affected side displayed on a monitor with a user device.
FIG. 10 shows an affected side image acquired by a user device.
FIG. 11 shows an affected side image from which an implant is removed through artificial intelligence image processing with respect to the affected side image of FIG. 10.
FIG. 12 shows an image in which the resolution and quality of an affected side image are improved.
FIG. 13 is an image obtained by changing a color of an image processed in FIG. 12 and turning it horizontally.
FIG. 14 is an image of which transparency is changed so that the image processed in FIG. 13 is transparent.
FIG. 15 is a view illustrating an additional process of improving image quality and visibility.
FIG. 16 is an image of finding the femoral head and the femoral shaft in the imaging-processed healthy side image.
FIG. 17 is an image for finding the femoral head and the femoral shaft in the imaging-processed affected side image.
FIG. 18 is an image obtained by overlaying a healthy side image of FIG. 16 and an affected side image of FIG. 17.
FIG. 19 shows an image in which a screw is operated after the rotation alignment is corrected.

### <Description of Reference Numerals>

10: Guide system for correcting rotational alignment of femur
100: Image capturing device
200: Image display device
300: User device
310: Image acquisition unit
320: Image quality improvement unit
330: Image registration unit
340: Result display unit

### Best Mode

The present invention may be variously changed and may have various forms, and specific embodiments will be illustrated in the drawings and described in detail in the text. However, this is not intended to limit the present invention to a specific disclosed form, and it should be understood that the present invention includes all modifications, equivalents, and substitutes included in the spirit and technical scope of the present invention. Similar reference numerals were used for similar components while describing each drawing.

Unless otherwise defined, all terms used herein, including technical or scientific terms, have the same meaning as those generally understood by those of ordinary skill in the art to which the present invention pertains. Terms such as those defined in commonly used dictionaries should be interpreted as having a meaning consistent with the meaning in the context of the related art, and are not interpreted as ideal or excessively formal meanings unless clearly defined in the present application.

Hereinafter, exemplary embodiments of the present invention will be described in more detail with reference to the accompanying drawings.

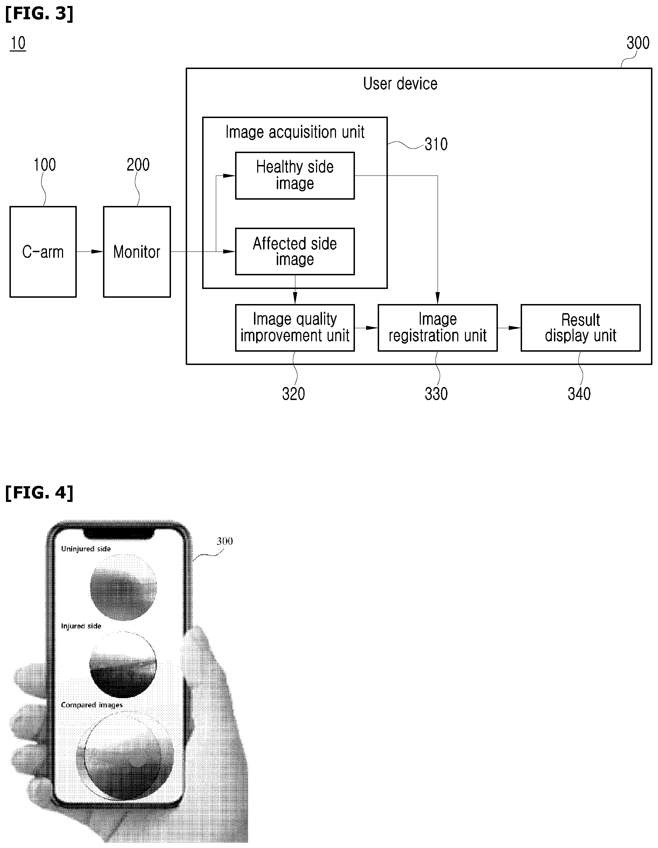

FIG. 2 is a diagram for explaining a guide system 10 for correcting rotational alignment of a femur using artificial intelligence image processing according to an embodiment of the present invention. FIG. 3 is a block diagram illustrating a guide system 10 for correcting rotational alignment of a femur using artificial intelligence image processing according to an embodiment of the present invention.

Referring to FIGS. 2 and 3, the guide system 10 for correcting rotation alignment of the femur using artificial intelligence image processing may include an image capturing device 100, an image display device 200, and a user device 300.

The image capturing device 100 may be a C-arm. The image display device 200 may be a monitor that displays an image captured by the C-arm. The C-arm is configured to capture X-ray images of a fractured femur (affected side) and a normal femur, healthy side, of a patient positioned on a table. Images of the affected side and the healthy side may be displayed on the monitor. An implant that combines the femoral fracture and the femur may appear in the affected side image (e.g., a left femur image). A femur image (e.g., a right femur image) that is not fractured may be displayed on the healthy side image.

The user device 300 may guide rotation alignment correction by comparing the healthy side image and the affected side image displayed on the monitor in the operating room. To this end, the user device 300 may capture the affected side image and the healthy side image displayed on the monitor or obtain the affected side image and the healthy side image through communication. The user device 300 may be a device capable of processing an image, such as a smartphone, a tablet, a virtual reality device (VR), an augmented reality device (AR), or the like. The user device 300 may include an image acquisition unit 310, an image quality improvement unit 320, an image registration unit 330, and a result display unit 340.

FIG. 4 illustrates an example of a user device 300 in which an image obtained by photographing an image displayed on a monitor is displayed.

Referring to FIG. 4, the image acquisition unit 310 may acquire an affected side image and a healthy side image by capturing the affected side image and the healthy side image displayed on the monitor, respectively. Alternatively, the image acquisition unit 310 may acquire the affected side image and the healthy side image from the affected side image and the healthy side image through communication with the monitor or the image capturing device. The user device 300 may photograph by fixing the monitor at a preset position, or may photograph an affected side image and a healthy side image displayed on the monitor while the user holds it in his or her hand according to a situation. For example, a smartphone may be used as the user device 300, and the camera module of the smartphone may acquire the affected side image and the healthy side image by photographing the affected side image and the healthy side image displayed on the monitor as the image acquisition unit 310, respectively. The acquired affected side image and the healthy side image may be used by the user in the operating room to guide the rotation alignment of the femur.

The image quality improvement unit 320 may remove the implant coupled to the femur from the acquired affected side image and improve the quality of the image such as sharpness. The image quality improvement unit 320 may use artificial intelligence image processing technology that extracts only the femur from the C-arm image (the affected side image, the healthy side image) to improve readability. For example, artificial intelligence software may be used to remove implants (e.g., a nail, a plate, or the like) unnecessary for image comparison from an affected side image displayed on a smartphone screen from the affected side image. In this case, when the user selects the implant using a finger, a touch pen, a cursor, or the like in the image on the affected side, the artificial intelligence software provided in the image quality improvement unit 320 may recognize the implant, separate the implant from the femur, and delete the implant. Alternatively, artificial intelligence software may be trained to distinguish between bones and implants and automatically separate and delete them. That is, the image quality improvement unit 320 may extract only the femur from the affected side image, remove the implant, and then perform high-resolution, high-clarity, and low-noise processing to improve readability of the affected side image. A process in which the image quality improvement unit removes the implant from the affected side image, restores the image of the bone, and improves the image quality will be described as an example below.

The image quality improvement unit 320 may first perform image preprocessing in order to remove a specific portion of the image such as an implant from the affected side image. Before starting the implant removal process, treatment can be performed to make the element under work as visible as possible. Then, the image is converted into a grayscale. Most medical images are grayscale and may not be necessary, but if the image is color, converting to grayscale is easier for imaging processing. Next. Reduce noise. Since medical images may be noisy, applying a noise reduction filter such as a Gaussian filter may soften the image and better distinguish bones from implants. A threshold value may be used for this distinction. The threshold value is a method of dividing an image into several parts according to pixel intensity. Since metal implants generally appear brighter (higher intensity) than bone, threshold techniques may help to separate these regions. For example, when using an adaptive threshold, it may help to distinguish between an implant and a bone by considering the neighborhood of a pixel and then finding an optimal threshold for each neighborhood. Thereafter, the region-based segmentation may be performed. If a threshold image is present, the implant can be identified using region-based segmentation. Then, the outline is found. You can identify the boundaries of the implant by finding the outline. Thus, an implant region is identified. Implant regions may be manually selected or identified using artificial intelligence software using machine learning models using characteristics such as region, shape, and strength. By this process, the identified and distinguished implant images can be removed. Thereafter, the implant region removed by the adjacent pixels may be filled. As an example of such a filling method, the identified region may be filled with values of adjacent pixels, such as smoothing the region using an interpolation method. In some cases, the basic bone structure can be reconstructed. This can be done using advanced methods such as morphological techniques or inpainting. After performing the process of removing and restoring the implant as described above, image post-processing may be performed. Additional post-processing steps such as contrast adjustment or additional noise reduction may be required to improve image quality after removal of the implant. Finally, the image can be reviewed and adjusted using image editing software to correct inaccuracies.

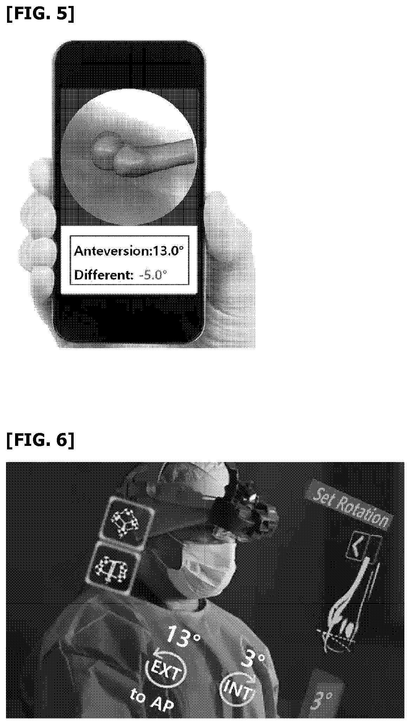

FIG. 5 is a diagram illustrating a result of registration displayed by the user device 300.

The image registration unit 330 may guide rotation alignment correction using the healthy side image and the affected side image. For example, the image registration unit 330 may overlay the affected side image on the healthy side image, match the size and position of the femur, and calculate the difference in angle. The process of such registrations is important in medical imaging, especially with respect to operations requiring alignment of various anatomical structures. In the case of femoral fracture surgery, it is essential for successful surgery to adjust the exact angle or position by matching the fractured surface with the normal surface. An example of a specific operation process of the image registration unit 330 is as follows. The image registration unit 330 may first perform image preprocessing so that the image is suitable for registration. In addition, the image registration unit 330 may perform image enhancement by applying the above-described techniques such as edge enhancement, noise reduction, and contrast adjustment. Thereafter, when the resolution is different between the affected side image and the healthy side image, resampling may be performed at the same resolution. Thereafter, the image may be cut or masked to focus only on the region of interest (e.g., the femur). The image registration unit 330 may also detect main features of the two images to be used to align the healthy side image and the affected side image. Specifically, a method such as a scale-invariant feature transform (SIFT) or a speeded-up robust features (SURF) may be used to detect a main function in two images. In addition, the image registration unit 330 may match the features between the two images by using a method such as FLANN (Approximate Nearest Neighbors). After this process, the image registration unit 330 may determine a transformation for aligning the features of the two images. First, depending on the misalignment, a fixed (rotation and conversion), an affine (including scaling and shearing), or a more complex nonlinear transformation may be selected. The method such as the RANSAC algorithm is used to reliably estimate the transformation parameters. Thereafter, the image may be aligned by applying the transformation. Specifically, the fractured side image may be resampled to align with the normal side image using the expected transform. In addition, methods such as bicubic or trilinear interpolation can make the transformations smooth and continuous.

The result display unit 340 may display the result of the registration on the screen of the smartphone so that the user may recognize the result. The result of the registration may include an angle difference of the femur in the healthy side image and the affected side image. The user may perform correction by correcting the rotation angle of the affected side to correct this angle. That is, the correction guide function as described above of the user device may be used to correct the rotation angle before a final process such as fixing the implant. Accordingly, the invention is accurate and quick in correcting the rotational alignment of the femur, which is good for both patients and doctors, and remarkably reduces the amount of X-ray exposure.

FIG. 6 shows an AR or VR device as another example of a user device.

In the guide system 10 for correcting the rotational alignment of the femur using artificial intelligence image processing of the present embodiment, in addition to the above-described smartphone as a user device, an MR-based (AR/VR) surgery guide device as illustrated in FIG. 6 may be adopted. For example, it is possible to improve visibility and surgical convenience and maximize registration accuracy by implementing AR in real time in an operating room. In conjunction with such an MR-based (AR/VR) surgical guide device, Femur may be extracted from the C-arm image using the bone alignment guide marker in real time, a 3D model may be constructed, and then the 3D model registration may be performed using the same. As a result, radiation free (X-ray free) surgery may be directed.

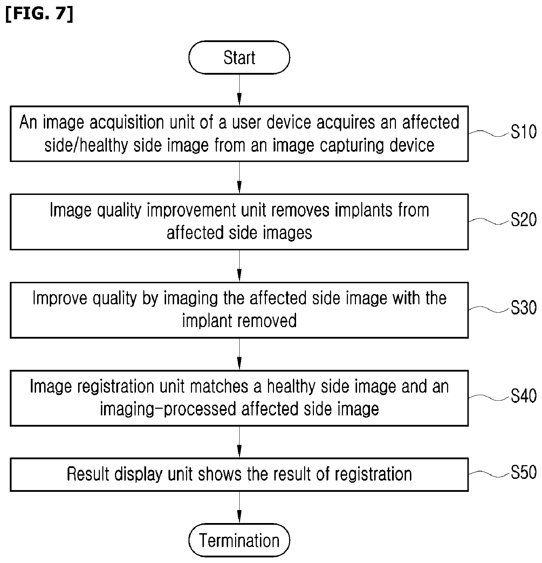

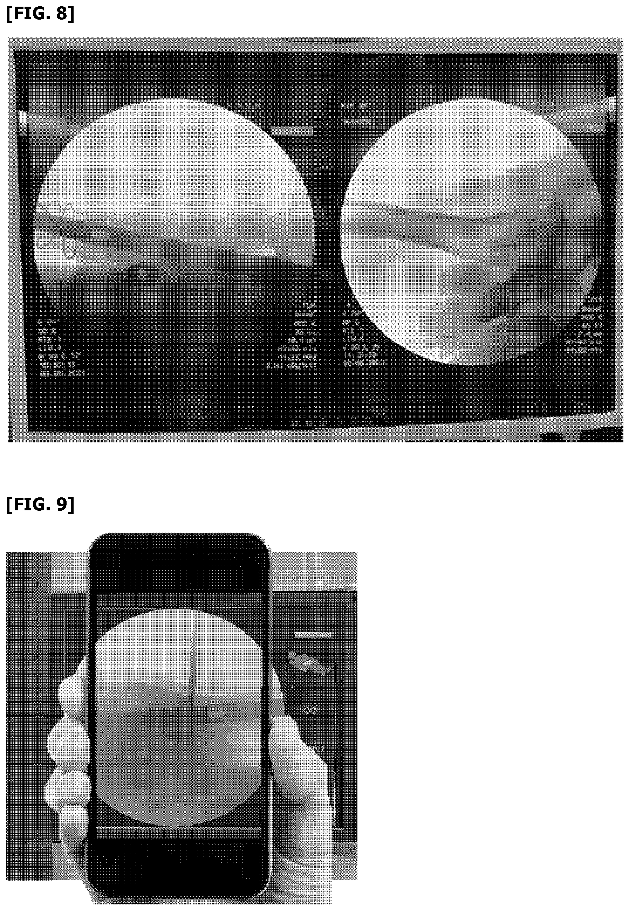

FIG. 7 is a flowchart illustrating a guide method for correcting rotation alignment of a femur using artificial intelligence image processing according to an embodiment of the present invention. FIG. 8 illustrates an example of a monitor displaying a C-arm image. FIG. 9 shows an image of an affected side obtained by photographing an image of the affected side displayed on a monitor with the user device 300.

In the guide method for correcting the rotation alignment of the femur using artificial intelligence image processing, first, the user device 300 acquires the C-ARM images of the healthy side and the affected side (S10). For example, prior to the operation, the above-described image capturing device 100 performs C-arm Lateral imaging of the healthy side and the affected side. According to the surgical procedure, after nail insertion, it can proceed to the stage before final fixation.

Referring to FIG. 8, in this state, an affected side image and a healthy side image may be displayed on a monitor (image display device 200). In this state, the user device 300 may obtain the healthy side image and the affected side image by photographing the C-ARM images (the affected side image and the healthy side image) of the healthy side and the affected side. That is, as illustrated in FIG. 9, when the user device 300 is a smartphone, the user device may obtain an affected side image and a healthy side image by photographing a monitor with a smartphone.

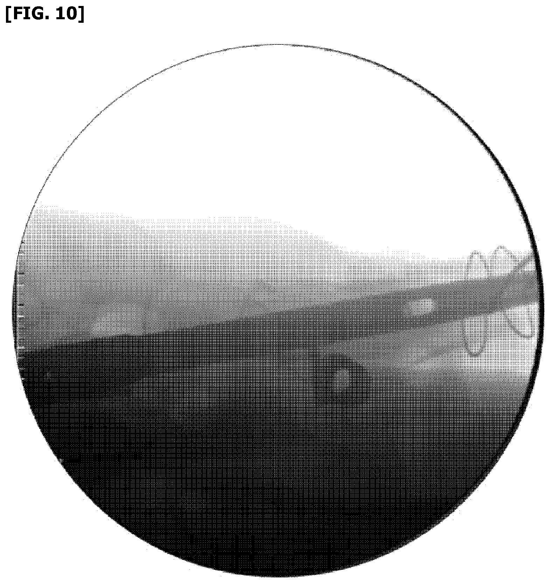

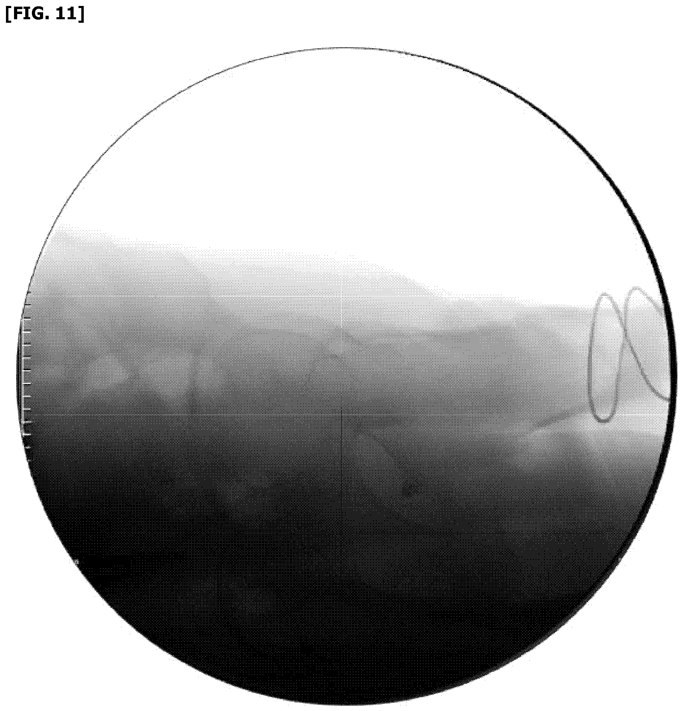

FIG. 10 shows an affected side image acquired by the user device 300. FIG. 11 shows an affected side image from which an implant is removed through artificial intelligence image processing with respect to the affected side image of FIG. 10.

Thereafter, as shown in FIGS. 10 and 11, the implant is removed from the affected side image (S20). Artificial intelligence software for restoring image distortion that occurs when a C-arm monitor image (an affected side image, a healthy side image) is photographed with the user device 300 may be applied. Such restoration may include both restoration of distortion, such as removing noise generated when photographing with the user device 300, and restoration of the original image in the area from which the implant is removed. For example, for effective image comparison, implants such as plates or nails in the affected side image are removed. The operation method of the artificial intelligence software for such imaging processing is as described above. For example, the image quality improvement unit 320 may delete the implant with a program such as a magic eraser for the affected side image shown in FIG. 10 and restore the shape of the femur of the portion from which the implant is removed as shown in FIG. 11.

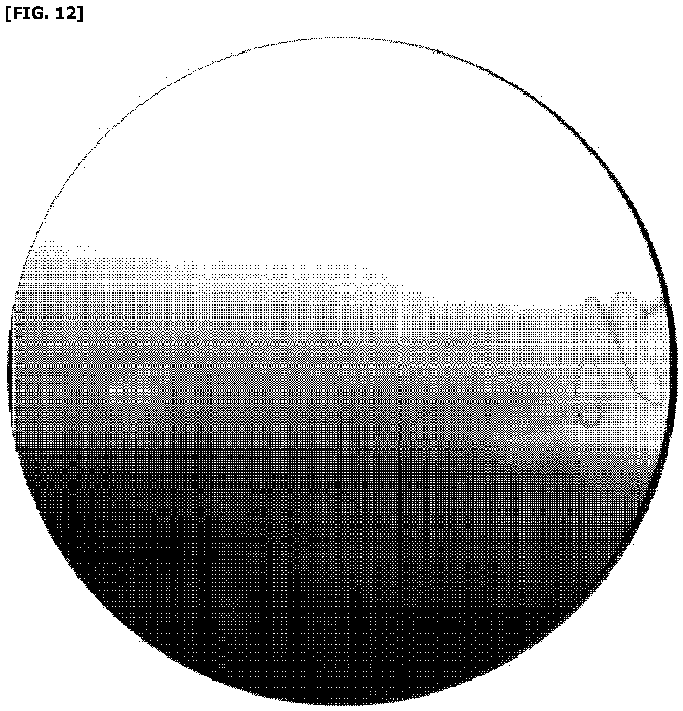

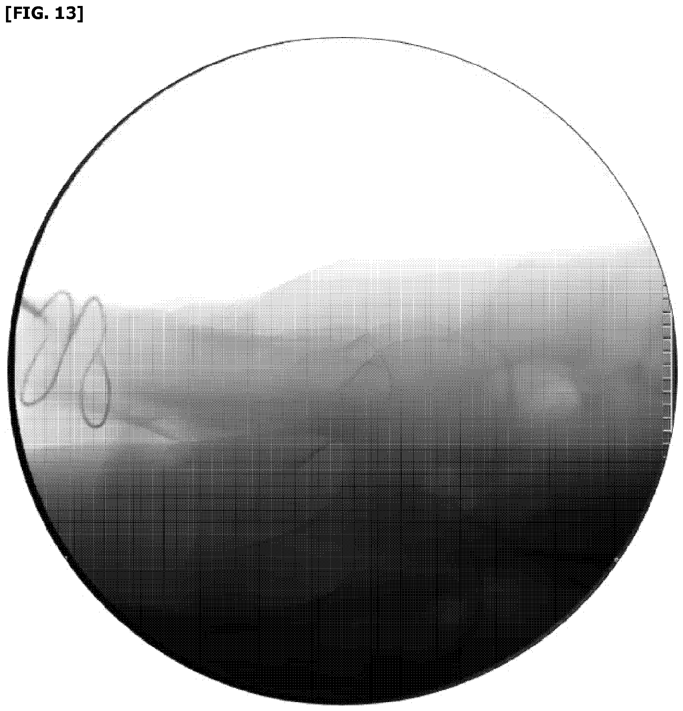

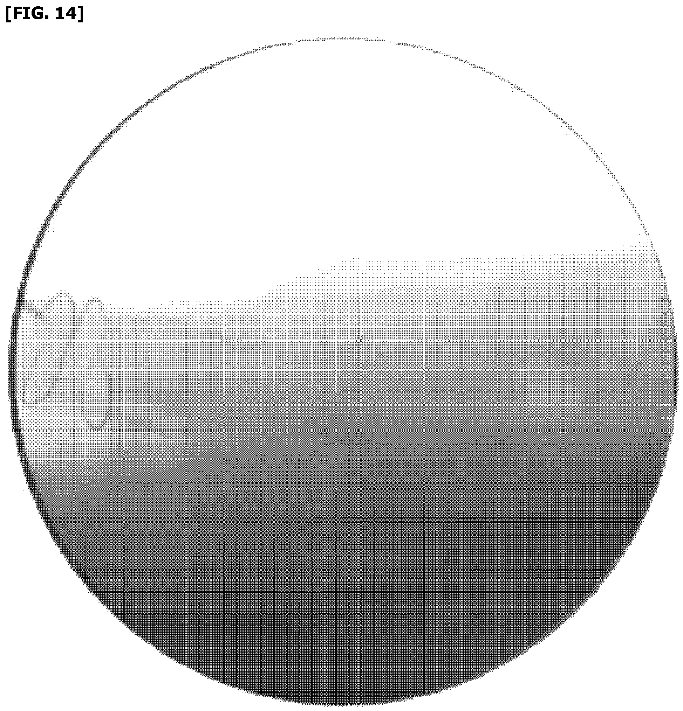

FIG. 12 shows an image in which the resolution and quality of an affected side image are improved. FIG. 13 is an image obtained by changing a color of an image processed in FIG. 12 and turning it horizontally. FIG. 14 is an image of which transparency is changed so that the image processed in FIG. 13 is transparent.

Next, the quality is improved by performing imaging treatment on the affected side image from which the implant has been removed (S30). As described above, even after the implant is removed from the affected side image, the image may not be clear yet. Therefore, an imaging process for improving image quality, such as resolution and sharpness, may be further performed on the affected side image from which the implant is removed by the image quality improvement unit 320. That is, in the subsequent registration process, imaging processing may be performed using software that may be made of at least a quality that may be compared with the healthy side image for comparison with the healthy side image.

For example, as illustrated in FIGS. 12 to 14, high-resolution, high-clarity, and low-noise processing may be performed on the image of the affected side from which the implant is removed. That is, high resolution processing may be performed on the affected side image from which the implant is removed as shown in FIG. 12, high clarity work may be performed as shown in FIG. 13, and low noise processing may be performed through transparency processing as shown in FIG. 14. This process may be performed by the image processing artificial intelligence software applied at the time of implant removal described above.

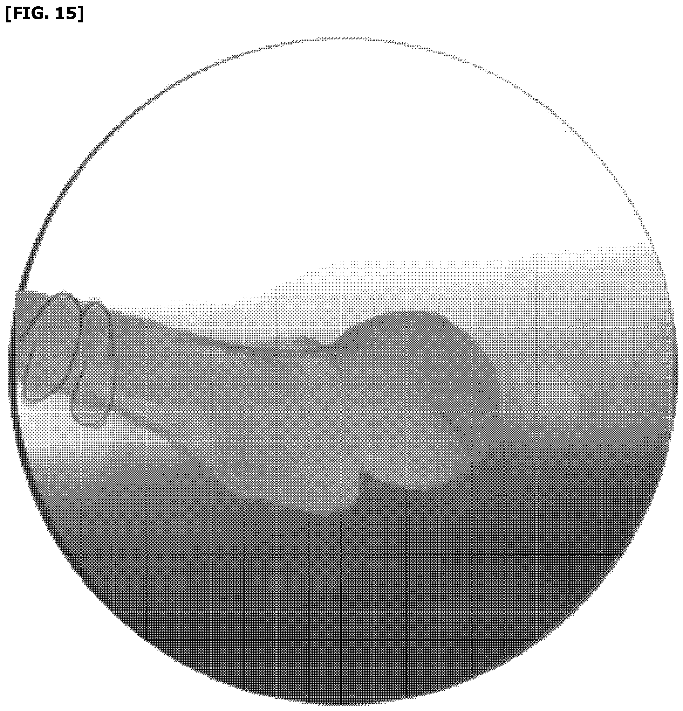

FIG. 15 additionally shows a process of improving the quality and visibility of an image.

After the above-described imaging process, as shown in FIG. 15, additionally, the quality and visibility of the image may be improved so that only the bone may be seen.

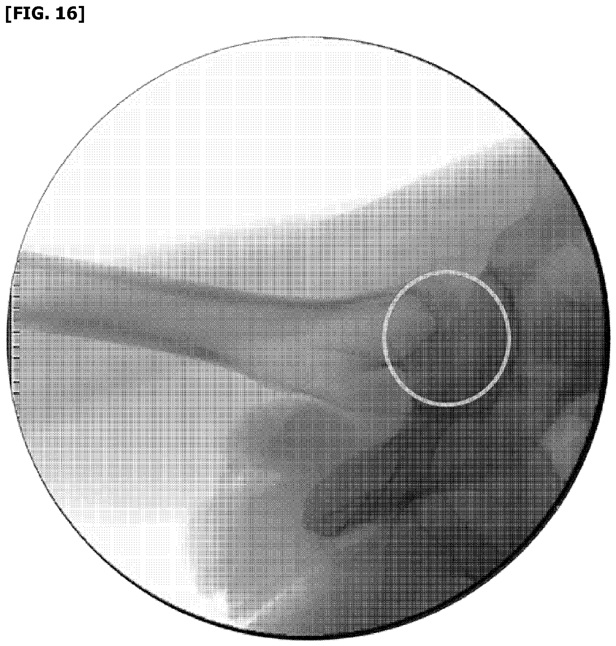

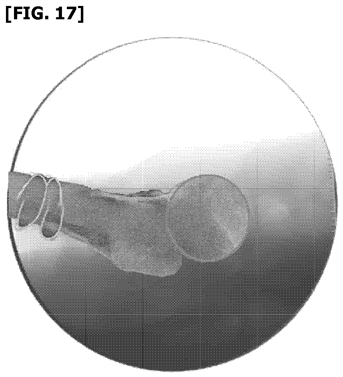

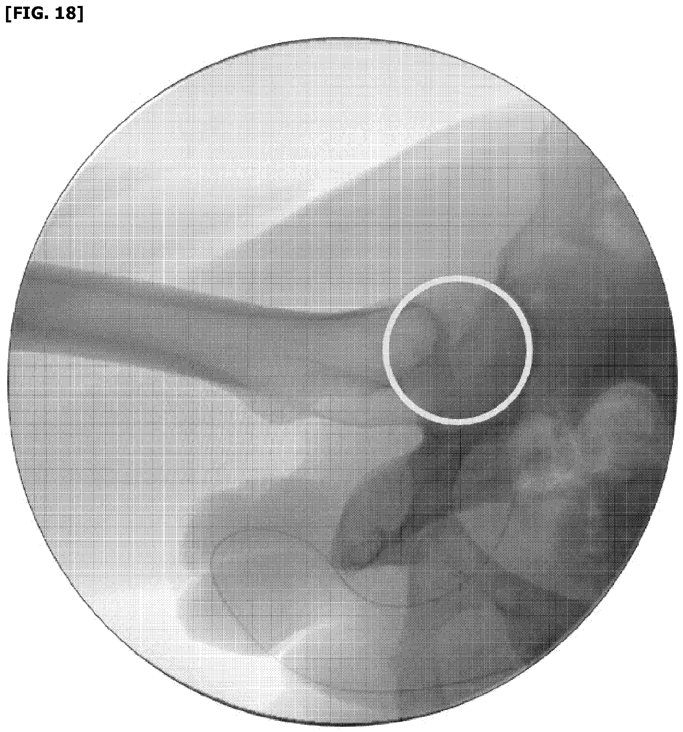

FIG. 16 is an image of finding the femoral head and the femoral shaft in the imaging-processed healthy side image. FIG. 17 is an image for finding the femoral head and the femoral shaft in the imaging-processed affected side image. FIG. 18 is an image obtained by overlaying a healthy side image of FIG. 16 and an affected side image of FIG. 17.

After improving the image quality as described above, the image registration unit 330 may match the healthy side image and the imaging-processed affected side image (S40). By removing implants from the affected side image, improving image quality, and then overlaying it on the healthy side image, it is possible to compare the scale, angle, and position. For example, the femoral head and the femoral shaft are found in the healthy side image and the imaging-processed affected side image. Thereafter, the image-processed affected side image on the healthy side image may be superimposed with the location and scale to be compared. As described above, when the affected side image is processed to be transparent and superimposes with the healthy side image, the femoral head and the femoral shaft may be visually recognized in the healthy side image, and this may be visually adjusted to match the femoral shaft of the affected side. In the operating room, it is necessary to take a C-arm image with the image capturing device 100 to compare the healthy side and the affected side, and to check whether the rotation angles of both sides are in the same state when viewed in the shape of the foot. However, in the past, it was difficult to determine whether the rotation angle was exactly the same simply by looking at the affected side and healthy side images displayed on the monitor. In the present embodiment, the image registration unit 330 of the user device overlays and matches the affected side image and the healthy side image. That is, it is possible to match the position and size of the femoral shaft of the healthy side and the femoral shaft of the affected side, and extract the difference in rotation angle. It can be used as a guide to correct the rotation angles of the affected side and the healthy side to match by using the difference in the rotation angles extracted in this way.

Next, the result of the registration may be displayed through the user device 300 (S50).

The femur may be extracted and a 3D model may be constructed using the affected side image and the healthy side image captured using the smartphone. Through the analysis of the registration result by the image registration unit 330 through the smartphone, the angle difference between the current anteversion and the healthy side may be displayed (see FIG. 5). As a result of Anteversion comparison through CT after surgery, very good correction was obtained with less than 3 degrees.

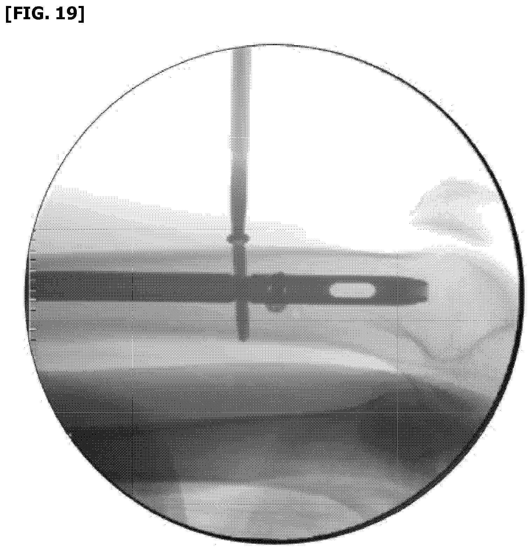

FIG. 19 shows an image in which a screw is operated after the rotation alignment is corrected.

As described above, after the rotation angle is corrected by the rotation alignment correction, the implant is fixed with a screw to complete the correction operation. Finally, the operation is completed after checking the alignment state.

The guide system and the method for correcting the rotational alignment of the femur using the artificial intelligence image processing according to the embodiment of the present invention may easily implement and use functions through a smartphone application. Accordingly, a separate complex system is unnecessary, and can be used simply and quickly in the form of an anteversion calculation app, so that a rotation angle correction guide can be provided very conveniently and accurately.

According to an embodiment of the present invention, provided are a system and a method for guiding position and size matching and correction for photographing a C-arm monitor image to restore distortion and reduce noise, to extract femur and improve image quality for improving readability of the image, and to compare a healthy side image and an affected side image.

The description of the present invention is for illustrative purposes, and those of ordinary skill in the art to which the present invention belongs will understand that the present invention can be easily modified into other specific forms without changing the technical spirit or essential features of the present invention. Therefore, it should be understood that the embodiments described above are exemplary in all aspects and are not limited. For example, each component described as a single type may be distributed and implemented, and similarly, components described as being distributed may be implemented in a combined form.

The scope of the present invention is indicated by the claims to be described later, and it should be interpreted that all changes or modified forms derived from the meaning and scope of the claims and the equivalent concept thereof are included in the scope of the present invention.

### Industrial Applicability

According to the present invention, provided are a guide system for correcting the rotational alignment of the femur, which guides the correction of the rotational alignment capable of converting a photographed C-arm monitor image into an original form without distortion and noise through artificial intelligence image processing technology for improving readability by extracting only the femur from a C-arm image, and a method thereof, thereby being more effective in the medical field.

## Claims

1. A guide system for correcting rotation alignment of a femur using artificial intelligence image processing, the guide system comprising:
an image capturing device configured to photograph an affected side and a healthy side of a patient;
an image display device configured to display an affected side image including a femur to which an implant photographed by the image capturing device is coupled and a healthy side image including a normal femur; and
a user device configured to obtain an affected side image and a healthy side image from the affected side image and the healthy side image, match the affected side image with the healthy side image based on the femur through artificial intelligence image processing, and calculate a rotation angle difference between a fractured femur in the affected side image and a normal femur in the healthy side image, thereby guiding rotation alignment correction of the affected side.

2. The guide system for correcting rotation alignment of a femur using artificial intelligence image processing of claim 1,
wherein the image capturing device is a C-arm using an X-ray,
the user device captures the affected side image and the healthy side image displayed on the image display device or acquires the affected side image and the healthy side image through communication, and the user device includes:
an image acquisition unit that acquires the affected side image and the healthy side image from the affected side image and the healthy side image, respectively;
an image quality improvement unit that removes an implant coupled to a femur from the affected side image acquired through the artificial intelligence image processing and improves the quality of an image including clarity thereof;
an image registration unit that overlays the affected side image and the healthy side image, matches the size and position of the femur, and calculates an angle difference of rotation alignment; and
a result display unit that displays the result of the alignment by the image registration unit so that a user can recognize the result.

3. The guide system for correcting rotation alignment of a femur using artificial intelligence image processing of claim 1,
wherein the user device is an MR-based (AR/VR) device, and is configured to extract femur from the affected side image and the healthy side image displayed on the image display device by using artificial intelligence image processing, configure a 3D model, and then perform 3D model matching by using the 3D model.

4. A guide method for correcting rotational alignment of a femur using artificial intelligence image processing, the guide method comprising:
acquiring, by an image acquisition unit of a user device, an affected side image and a healthy side image from an affected side image and a healthy side image by an image capturing device;
removing, by an image quality improvement unit, an implant from the affected side image through artificial intelligence image processing;
improving, by the image quality improvement unit, quality by imaging the affected side image from which the implant is removed;
matching, by an image registration unit, the healthy side image and the imaged affected side image; and
displaying, by a result display unit, a result of the registration including an angle difference between rotational alignment of the affected side femur and the healthy side femur calculated as a result of the registration.

5. The guide method for correcting rotational alignment of a femur using artificial intelligence image processing of claim 4, wherein the removing of the implant from the affected side image comprises:
restoring image distortion that occurs when the image quality improvement unit obtains the affected side image and the healthy side image through artificial intelligence image processing;
separating the femur and the implant from the affected side image and removing the implant through artificial intelligence image processing by the image quality improvement unit; and
restoring an original femur image to a region from which the implant is removed by the image quality improvement unit.

6. The guide method for correcting rotational alignment of a femur using artificial intelligence image processing of claim 5, wherein the imaging of the image of the affected side image to improve quality comprises:
performing high resolution processing to improve resolution of the affected side image from which the implant is removed;
performing a high sharpness operation to improve sharpness of the affected side image from which the implant is removed; and
performing low noise processing through transparency on the high resolution processing and the affected side image on which the high sharpness operation has been performed.

7. The guide method for correcting rotational alignment of a femur using artificial intelligence image processing of claim 6, wherein the matching of the healthy side image and the imaged affected side image comprises:
finding a femoral head and a femoral shaft in the healthy side image and the imaged affected side image, respectively;
matching the imaged affected side image with the healthy side image by superimposing the position of the femoral shaft with the scale of the femoral head; and
calculating an angle difference between rotation alignment of the femoral head of the healthy side and the femoral head of the affected side.
